# EUROPÄISCHE PATENTSCHRIFT

(11) **EP 3 522 928 B1**
(45) Veröffentlichungstag und Bekanntmachung des Hinweises auf die Patenterteilung: **22.12.2021**
(21) Anmeldenummer: 17791949.5
(22) Anmeldetag: 06.10.2017
(51) Int. Cl.: A61K 47/10, A61K 47/12, A61K 47/34

(54) **TRANSLOKATION VON SYNTHETISCHEN POLYMEREN DURCH LIPIDMEMBRANE**
TRANSLOCATION OF SYNTHETIC POLYMERS BY LIPID MEMBRANES
TRANSLOCATION DE POLYMÈRES SYNTHÉTIQUES À TRAVERS UNE MEMBRANE LIPIDIQUE

(30) Priorität: 07.10.2016 DE 102016119102
(43) Veröffentlichungstag der Anmeldung: 14.08.2019
(73) Patentinhaber: Forschungszentrum Jülich GmbH, 52425 Jülich (DE)
(72) Erfinder: FLEURY, Jean Baptist, 66123 Saarbrücken (DE)
(74) Vertreter: Fitzner, Uwe
(86) Internationale Anmeldenummer: PCT/EP2017/075496
(87) Internationale Veröffentlichungsnummer: WO 2018/065583

(56) Entgegenhaltungen:
- WO-A1-01/40429
- WO-A1-97/15287
- WO-A2-2004/096140
- US-A1- 2005 277 739

## Beschreibung

Die vorliegende Erfindung betrifft ein Copolymer enthaltend alternierende hydrophobe und hydrophile Einheiten sowie die Verwendung dieser Copolymere als Träger für Wirkstoffe durch eine Doppellipidschicht, insbesondere Lipidmembran. Copolymere, die in der Medizin verwendet werden und eine Trennmembran passieren können, müssen biokompatibel sein. Aus der WO 2015/161841 A2 sind modifizierte Poly(Dicarbonsäure-Multiol-Ester) zur kontrollierten Wirkstofffreisetzung bekannt, die als implantierbare Trägersysteme biokompatibel sind. Dabei werden Wirkstoffe an freie Hydroxylgruppen des Copolymers kovalent gebunden. Werner et al. (Biomacromolecules, 2015, 16, 125 ― 135) simuliert anhand von Rechenmodellen amphiphile Copolymere und versucht deren Eigenschaften bezüglich Absorption und passiver Translokation an oder durch eine Lipid-Bilayer-Membran theoretisch vorher zu sagen. Im Ergebnis sollten die Copolymere einen lipophilen Charakter haben mit HLB-Wert zwischen 5 und 10. *Werner et* al. beschreiben außerdem keine chemischen Struktur der simulierten Copolymere.

Im Sinne der Erfindung wird der Begriff "Translokation" durch eine Lipid-Bilayer-Membran als Durchdringen dieser Membran verwendet, bevorzugt durch passiven Transport, also den Standortwechsel von Molekülen, insbesondere Copolymeren, von einer Seite dieser Membran auf die andere, unabhängig von dem Mechanismus. Wie von *Werner et al.* beschrieben, sind z.Z. einige Stoffe bekannt, die durch Translokation in Zellen eindringen, darunter z.B. sog. CPP (cell-penetrating peptides), Polyelektrolyte. Als weitere Trägersysteme zur kontrollierten Freisetzung werden in der WO 2015/161841 z.B. nicht-wasserlösliche Mikro- und Nanopartikel erwähnt. Block-Copolymere, Statistische Copolymere oder Gradient-Copolymere sind beispielsweise aus der DE 69 620 898 T2, WO 01/70288 A2, WO 2005/121196 A1 oder WO 2007/109584 A1 bekannt. Trägermoleküle für z.B. Wirkstoffe gegen Krebs sind auch aus der WO 2008/150996 A1 bekannt. Eine Translokation wird diesen Trägern jedoch nicht zugeordnet.

Ziel dieser Trägersysteme zur kontrollierten Wirkstofffreisetzung (auch Drug Carrier Systems genannt) ist es einerseits, ein schnelles Ausscheiden von Wirkstoffen aus dem Körper zu verhindern. Die meisten konventionellen Wirkstoffe sind klein und verweilen deshalb nur kurz im Blutkreislauf, da sie aufgrund ihrer Größe unterhalb der Nierenschwelle liegen, und deshalb vom Blut getrennt und wieder ausgeschieden werden.

Andererseits sollen die Trägersysteme nach Möglichkeit die Wirkstoffe spezifisch in die Nähe bestimmter Organe oder Zellen transportieren und dort möglichst kontrolliert freisetzen. Als weiterer Stand der Technik kann WO 97/15287 genannt werden.

Aufgabe der vorliegenden Erfindung war es, Träger zur Verfügung zu stellen, die eine Translokation durch eine Doppellipidschicht, also Lipid-Bilayer-Membran, insbesondere

Zellmembran ermöglichen, und Nachteile des Standes der Technik - wie z.B.

Nanotoxizität oder unerwünschte Wechselwirkungen aufgrund des ionischen Charakters der Moleküle mit Zellkomponenten - nicht aufweisen.

Ferner sollen diese Moleküle auch nicht membrangängige Wirkstoffe und Mittel mit biologischer Wirkung in Zellen überführen bzw. transportieren können. In einer Ausführung soll die Zellmembran dabei nicht beschädigt werden.

Die erfindungsgemäßen Copolymere sollen außerdem in wässrigem Medium löslich sein, um sie unter physiologischen Bedingungen einzusetzen.

Gelöst wird diese Aufgabe durch ein Copolymer kovalent oder physikalisch mit mindestens einem Wirkstoff verbunden wie in Anspruch 1 beschrieben, enthaltend oder bestehend aus einer Hauptkette enthaltend alternierende hydrophobe und hydrophile Einheiten. Das Copolymer weist bevorzugt einen LCST-Wert von 20°C bis 80°C, 20 °C bis 70°C auf-, mehr bevorzugt 25°C bis 90°C, insbesondere 25°C bis 90°C oder 35°C bis 90°C.

Die Hauptkette ist das Polymerisationsprodukt, gegebenenfalls Kondensations- oder Additionsprodukt der mindestens zwei Monomere, die das Copolymer bilden und liegt im Wesentlichen linear, bevorzugt linear vor.

Erfindungsgemäß beschreibt der LCST-Wert die untere kritische Lösungstemperatur, d.h. unterhalb dieses LCST-Wertes sind die erfindungsgemäßen Copolymere in Wasser oder wässrigen Lösungen vollständig löslich. Bei Temperaturen oberhalb des LCST-Wertes werden die Polymere unlöslich.

LCST lässt sich durch Turbidimetrie oder durch dynamische Differenzkalorimetrie bestimmen Der LCST-Werte wird alternativ visuell bestimmt; dabei ist der LCST-Wert jene Temperatur bei welcher eine Trübung der Lösung auftritt. Diese wird durch das Ausfallen der Polymere verursacht.

In einer Alternative besitzen die Polymere keinen genauen, punktuellen LCST-Wert sondern einen LCST-Bereich. Erfindungsgemäß betreffen die LCST-Werte und/oder insbesondere im Falle von LCST-Bereichen ein Temperatur-Intervall um den bestimmten Wert von 30 oder 20% des jeweiligen Wertes, bevorzugt von 15%, insbesondere 10%. Dies bedeutet: ein zum Beispiel mit 20°C bestimmter LCST-Wert beinhaltet auch Werte von 14 bis 26 oder 16 bis 24, bevorzugt 17 bis 23, insbesondere 18 bis 22 jeweils Grad Celsius.

Vollständig löslich bedeutet, dass die erfindungsgemäßen Copolymere molekular dispers gelöst sind, also eine echte Lösung bilden. In diesem Zusammenhang wird der Begriff "wässrige Lösung" für Lösungen verwendet, die neben Wasser als Lösungsmittel noch weitere molekular dispers gelöste Stoffe, wie z.B. Salzionen enthalten können. Mit Blick auf Körperflüssigkeiten enthalten wässrige Lösungen auch weitere Stoffe, die suspendiert oder dispensiert vorliegen können.

Die erfindungsgemäßen Polymere bestehen aus alternierenden hydrophilen und hydrophoben Einheiten. Im Sinne der Erfindung sind die einzelnen Einheiten der erfindungsgemäßen hydrophob-hydrophil-alternierenden Copolymere nicht identisch mit den als Ausgangsstoff verwendeten Monomeren, insbesondere ist der hydrophile bzw. hydrophobe Charakter der Einheiten der erfindungsgemäßen Polymere nicht in allen Ausführungen identisch mit den hydrophilen bzw. hydrophoben Charakter der Monomere, die als Ausgangsstoffe eingesetzt werden.

Erfindungsgemäß ist eine hydrophobe Einheit und/oder Gruppe eine Einheit und/oder Gruppe die den LCST-Wert eines Polymers reduziert. Dies kann durch einfachen Vergleich bestimmt werden: zunächst wird der LCST-Wert eines synthetisierten oder Ausgangs-Polymers bestimmt. Anschließend wird ein Derivat dieses Polymers synthetisiert das eine weitere Einheit und/oder Gruppe enthält. Alternativ wird eine solche Einheit und/oder Gruppe in das Ausgangs-Polymer eingebaut. Anschließend wird der LCST-Wert dieses zweiten Polymers bestimmt. Ist der zweite gemessene LCST-Wert niedriger als der erste handelt es sich eine hydrophobe Einheit, ist der zweite gemessene LCST-Wert höher als der erste handelt es sich eine hydrophile Einheit. Somit ist der LCST Wert ein Maß für die Hydrophilie oder Hydrophobie eines Polymers.

Ein Beispiel für eine hydrophile Einheit ist EO beziehungsweise als Ausgangsstoff PEG. Beispiele für hydrophobe Einheiten beziehungsweise Ausgangsstoffe sind Polyalkandiol, Polypropylenglycol, Polybutylenglycol und/oder Polytetramethylenglycol für Alkohole.

So kann beispielsweise Glutarsäure zur Herstellung erfindungsgemäßer Polymere verwendet werden, obwohl sie mit 640g/L bei 20°C gut wasserlöslich ist. Glutarsäureester, wie sie in Polyestern vorliegen, sind jedoch im Wesentlichen hydrophob und allenfalls gering wasserlöslich. Beispielsweise beträgt die Löslichkeit von Glutarsäuredimethylester in Wasser nur 53g/L.

Entscheidend ist das Löslichkeitsverhalten des Polymers in Wasser.

Die erfindungsgemäßen Polymere besitzen eine untere kritische Lösungstemperatur (LCST). Unterhalb der LCST sind die Polymere löslich, oberhalb unlöslich. Durch Molekulargewichte der Ausgangsstoffe der hydrophilen und hydrophoben Einheiten sowie deren Molekurlargewichtsverhältnis lässt sich der LCST einstellen. Außerdem ist die Polarität der Komponenten entscheidend. Je unpolarer beispielsweise der Ausgangsstoff der hydrophoben Einheiten ist, umso geringer ist dessen Molekulargewicht um einen gegebenen LCST einzustellen. (Vgl. Tabelle 1: P(EG4PG3) vs P(C4EG4); bei P(C5EG4) wurde ein LCST-Wert von 13°C bestimmt). Außerdem ist die Natur der Bindung, die bei der Polymerisationsreaktion gebildet wird, entscheidend. Esterbindungen sind unpolar und vergrößern somit die hydrophoben Einheiten. Amid- oder Urethanbindungen sind polar und vergrößern somit die hydrophilen Einheiten.

Ein besonderer Vorteil der erfindungsgemäßen Copolymere ist ihre gute Wasserlöslichkeit unter physiologischen Bedingungen, insbesondere bei Temperaturen zwischen 25°C und 50°C, bevorzugt 25°C bis 45°C oder 30°C bis 50°C.

Die Copolymere sind aufgrund ihrer LCST-Werte hydrophil, haben mithin einen hydrophilen Gesamtcharakter, d.h. sie sind bei Raumtemperatur wasserlöslich.

Im Sinne der Erfindung bedeutet "alternierende hydrophobe und hydrophile Einheiten", dass alternierende Copolymere vorliegen, in denen die hydrophoben und hydrophilen Einheiten abwechselnd vorliegen: -abababab-. a symbolisiert beispielsweise eine hydrophobe und b eine hydrophile Einheit oder vice versa. Mithin unterscheiden sich diese alternierenden Copolymere von z.B. statistischen Copolymeren, in denen die Verteilung der beiden Einheiten in der Kette zufällig ist, wie z.B. -aaabbababbbbaababaa-. Unterschiedlich sind auch Gradienten-Copolymere, die den statistischen Copolymeren ähnlich sind, in denen jedoch der Anteil einer Einheit im Verlauf der Kette zunimmt, während der Anteil der anderen Einheit abnimmt, wie z.B. -aaaabaaabbaabbbabbbb. Auch Block-Copolymere und Segment-Copolymere unterscheiden sich im Aufbau von den erfindungsgemäßen alternierenden Copolymere, da hier längere Sequenzen oder Blöcke aus jeweils den hydrophoben und hydrophilen Einheiten vorliegen.

Die erfindungsgemäßen Copolymere haben mithin ein Verhältnis von hydrophilen zu hydrophoben Einheiten von im Wesentlichen 1:1, mithin bezüglich des Begriffs "Wesentlich" eine Abweichung von jeweils maximal 10 %, also ein Verhältnis von 1.1 : 0.9 bis 0.9 : 1.1; bevorzugt 1:1. Abweichungen können sich insbesondere ergeben, wenn eine der beiden Monomereinheiten bei der Synthese im Überschuss verwendet wird, so dass diese Monomereinheit bevorzugt die Kettenenden bildet.

In einer Alternative haben die hydrophilen Einheiten (EO-)I oder Derivate wie zum Beispiel Polyetherdicarbonsäuren (wie auch im Folgenden beschrieben) ein höheres Molekulargewicht als die Hydrophoben Einheiten und/oder die hydrophoben Ausgangsstoffe.

Die erfindungsgemäßen Copolymere sind auch über ihre zahlenmittleren Molmassen Mn charakterisiert, von 2000 bis 50000 g/mol, bevorzugt 2500 bis 25000, besonders bevorzugt 3000 bis 14000 g/mol, von 3200 bis 13000, insbesondere 3500 bis 12000 g/mol.

In einer Alternative, die nicht beansprucht ist, ist die zahlenmittlere Molmasse Mn von 1000 bis 20000 bevorzugt von 1000 bis 10000, insbesondere 1000 bis 5000 oder 1000 bis 3000 g/mol.

In einer anderen Alternative ist die zahlenmittlere Molmasse Mn von 7000 bis 14000 , bevorzugt von 8000 bis 13000, insbesondere 7500 bis 12000 g/mol oder ein Molekulargewichtsbereich von mindestens 5000 bis unterhalb 10000 , also 5000 < Mn < 10000.

Gemäß der Formel A: D=Mw/Mn ergibt sich eine Polydispersität D für die erfindungsgemäßen Copolymere von 1,01 bis 3,0, bevorzugt 1,3 bis 2,0, bestimmt mittels geeigneter GPC-Methoden.

Bevorzugt besitzen die erfindungsgemäßen Copolymere einen Wert D von 1,01 - 1,6, insbesondere 1,3 - 1,6.

In einer Alternative, für fraktionierte Polymere ist D von 1,01 bis 1,2, oder 1,01 bis 1,5, bevorzugt 1,01 bis 1,3, besonders bevorzugt 1,01 bis 1,2, insbesondere 1,01 bis 1,17 oder 1,01 bis 1,15. Erfindungsgemäß bedeutet fraktioniert eine Trennung nach Molekulargewicht, das heißt es liegen Fraktionen mit enger Molekulargewichtsverteilung vor.

In einer Ausführung liegen die erfindungsgemäßen Copolymere als diskrete Moleküle vor, d.h. Sie sind nicht quer vernetzt und/oder bilden auch keine Gele.

In einer Alternative sind die jeweiligen Bausteine, also Monomerbausteine, mithin die hydrophoben und/oder hydrophilen Einheiten, Bausteine mit einer engen Molekulargewichtsverteilung. D.h., die verwendeten Edukte haben ebenfalls eine enge Molekulargewichtsverteilung, bevorzugt eine Polydispersität D wie oben angegeben. Die Bausteine können auch eine definierte Molmasse und molekulare Struktur aufweisen, beispielsweise Dicarbonsaeuren wie Butandisäure oder Hexandisäure.

In einer Ausführung ist das erfindungsgemäße Copolymer dadurch gekennzeichnet, dass es eine im Wesentlichen lineare Hauptkette enthält oder daraus besteht. Der Begriff "im Wesentlichen linear" beschreibt erfindungsgemäß ein Molekül mit einer linearen Hauptkette durch die die Länge des Copolymers bestimmt ist. Die erfindungsgemäßen Copolymere können gegebenenfalls Verzweigungen aufweisen, sind jedoch aus bifunktionalen monomeren Bausteinen aufgebaut die gegebenenfalls kurze Verzweigungen zu Seitenketten aufweisen. D.h. an die lineare Hauptkette, die die Basis darstellt, kann Seitenketten aufweisen. Bei den Seitenketten handelt es sich in einer Alternative um - unter physiologischen Bedingungen - inerte Reste wie z.B. Alkyl-, Aryl- oder Alkylarylreste.

In einer weiteren Alternative sind einige Seitenketten, die Polymerenden oder zusätzliche Seitenketten oder alle mit terminalen, funktionalen Gruppen versehen zur Bindung von Wirkstoffen. Dabei enthalten entweder die Copolymere oder die Wirkstoffe eine komplementäre funktionelle Gruppe A die jeweils mit der komplementären funktionellen Gruppe B reagiert, die im jeweils anderen Reaktionspartner enthalten ist. Die komplementären funktionellen Gruppe A und B werden ausgewählt aus der Gruppe enthaltend oder bestehend aus:

| | | |
|---|---|---|
| A | und | B |
| | oder | |
| B | und | A |
| -SH | | -C(O)-OH |
| -NH₂ | | -C(O)-O-C(O)- |
| -OH | | -NCO |
| -O-(CO)-NH-(CO)-NH₂ | | -NH-C(O)-OR |
| -O-(CO)-NH₂ | | -CH₂-OH |
| >NH | | -CH₂-O-R |
| | | -NH-CH₂-O-R |
| | | -NH-CH₂-OH |
| | | -N(-CH₂-O-R)₂ |
| | | -NH-C(O)-CH(-C(O)OR)₂ |
| | | -NH-C(O)-CH(-C(O)OR)(-C(O)-R) |
| | | -NH-C(O)-NR¹R² |
| | | > Si(OR)₂ |
| | | 1,2-Epoxyethyl |
| -C(O)-OH | | 1,2-Epoxyethyl |
| | | -N=C=N- |
| | | -C(O)-N(CH₂-CH₂-OH)₂ |
| Ethylengruppe (Vinylgruppen) | | Ethylengruppe |

In einer Ausführung sind die erfindungsgemäßen Copolymere nicht das Reaktionsprodukt einer radikalischen Polymerisation. In einer Alternative enthalten die erfindungsgemäßen Copolymere keine Doppelbindungen zur weiteren Vernetzung.

In einer weiteren Ausführung enthalten die erfindungsgemäßen Copolymere mindestens zwei hydrophile und zwei hydrophobe Einheiten, bevorzugt 3, besonders bevorzugt 5, insbesondere 10 jeweils hydrophile und hydrophobe Einheiten oder bestehen daraus.

In einer Ausführung ist das Copolymer im Wesentlichen nicht-ionisch. Der Begriff "im Wesentlichen nicht-ionisch" beschreibt erfindungsgemäß ein Molekül das aufgrund seines hydrophilen Charakters im Wässrigen löslich ist und nicht aufgrund eines Ionen-Charakters. Die Copolymere können terminale Endgruppen und/oder Seitengruppen aufweisen, die je nach pH als Ionen vorliegen. Diese beeinflussen jedoch nicht die Löslichkeit im Wässrigen.

Die hydrophile Einheit der Copolymere wird durch Formel

(I) -(CH2-CH2-O-)I

definiert, mit I= 2 bis 20, bevorzugt 2 bis 18 oder 2-16.

Eine Ausführung der vorliegenden Erfindung betrifft Copolymere mit I = 3-20, bevorzugt 3-10, insbesondere 4-6.

Das erfindungsgemäße Copolymer ist mithin in einer Alternative dadurch gekennzeichnet, dass mindestens vier, bevorzugt mindestens sechs Etherfunktionen und/oder Derivate davon in der Hauptkette vorliegen.

Im Sinne der Erfindung ist eine Etherfunktion eine funktionelle Gruppe, also eine Ethergruppe der allgemeinen Formel R1-O-Ra, wobei ist, Ra = oder R1 = CH2-CH2 oder R1 und/oder Ra unabhängig voneinander ein Rest mit C3 bis C10 Kohlenstoffkette ist. Der Rest kann linear oder verzweigt sein oder Aromate enthalten oder daraus bestehen.

In einer weiteren Ausführung ist das Copolymer dadurch gekennzeichnet, dass mindestens zwei, bevorzugt mindestens vier weitere Funktionen in der Hauptkette vorliegen, ausgewählt aus der Gruppe enthaltend oder bestehend aus Ester-, Amid- und/oder Urethan-Funktionen und/oder Derivate und/oder Kombinationen davon.

Eine weitere Ausführung der vorliegenden Erfindung betrifft das erfindungsgemäße Copolymer, welches ein Kondensationsprodukt aus Dicarbonsäuren und Diolen ist, und mithin mindestens 2, bevorzugt mindestens 4 Esterfunktionen und/oder Derivate davon in der Hauptkette aufweist.

Im Sinne der Erfindung ist eine Esterfunktion eine funktionelle Gruppe, also eine Estergruppe der allgemeinen Formel R2COOR3. Mithin sind die Monomere des erfindungsgemäßen Copolymers über Estergruppen in der Hauptkette miteinander verknüpft.

Das erfindungsgemäße Copolymer mit Esterfunktion wird durch die allgemeine Formel II dargestellt: (-R2-CO-O-R3-O-CO-)n;
mit n = 3 - 500, bevorzugt 3 - 100 wobei die angegeben Mw Werte nicht überschritten werden und
wobei R2 und R3 unabhängig voneinander 1 bis 60 (wegen je 30 EO-Einheiten) C-Atome enthalten oder daraus bestehen und gegebenenfalls mindestens ein Sauerstoff-Atom in der Hauptkette zwischen zwei C-Atomen enthalten.

R2 ist der Rest der Dicarbonsäure und R3 ist der Rest des Diols. Der Rest kann linear oder verzweigt sein oder Aromate enthalten oder daraus bestehen.

Die Dicarbonsäure weist 4-12 C-Atome, bevorzugt 4-8, besonders bevorzugt 4-6 auf.

In einer Ausführung enthält R2 und/oder R3 mindestens eine hydrophile Einheit gemäß

Formel I oder besteht daraus.

In einer Alternative ist das erfindungsgemäße Copolymer mithin das Kondensationsprodukt aus Dicarbonsäure der Formel II ― 1a: HOOC-R2-COOH mit einem Polyethylenglycol (PEG),
der Formel II-1b: HO-(EO-)I-H, R2 und I wie oben beschrieben. E steht für CH2-CH2, also zwei CH2-Gruppen, EO steht mithin für Ethoxy.

Falls R2 mindestens 10 C-Atome, bevorzugt als Kette enthält, ist R3 aus maximal 20 EO-Einheiten aufgebaut.

Bevorzugt wird PEG eingesetzt mit einem Molekulargewicht von 150-1000 g/mol, bevorzugt ca. 150 bis 600 g/mol, insbesondere 200 bis 450 oder 200 bis 300 g/mol. In dieser Alternative ist R3 ein Rest aus 2-30 EO-Gruppen, bevorzugt 4-20, besonders bevorzugt 6-14, 4-10 oder 4-6.

In einer weiteren Alternative ist das erfindungsgemäße Copolymer das Kondensationsprodukt einer Polyethylglycoldicarbonsäure der Formel II-2a:

HOOC-CH2-O-(EO-)(I-2)-CH2-O-COOH

mit einem Diol der Formel II-2b:

HO-(CH2)n1-OH,

n1 = 2 - 20, bevorzugt 2-10, besonders bevorzugt 2-8, 2-6 oder 2-4.

Das erfindungsgemäße Copolymer weist in einer Ausführung mindestens zwei, bevorzugt vier Funktionen in der Hauptkette auf die keine Esterfunktion sind.

Im Sinne der Erfindung sind in einer Alternative diese Funktonen Amidfunktionen. Eine Amidfunktion ist eine funktionelle Gruppe, also ein Amid der Formel R4-CO-NRb2-R5. D.h. eine Amidfunktion ist das Produkt einer Kondensation einer Carbonsäure mit einem Amin.

Rb ist unabhängig voneinander H, -CH3, -CH2CH3,-CH2CH2CH3,-CH2CH2CH2CH3, bevorzugt Methyl, Alkyle mit C4-C8-Ketten, Aryle (bevorzugt Phenyl oder Naphthyl), Alkylaryle (bevorzugt Benzyl) oder andere inerte Reste, besonders bevorzugt H, Methyl oder Ethyl, insbesondere H oder Methyl.

Mithin sind die Monomere des Copolymers über Amidfunktionen miteinander verknüpft.

Das erfindungsgemäße Copolymer mit Amidfunktion wird durch die allgemeine Formel III dargestellt: -(R4-CO-NRb-R5-NRb-CO-)m;
m = 5 ― 500, bevorzugt 5-100 wobei die angegeben Mw Werte nicht überschritten werden und
R4 ist der Rest der Dicarbonsäure und R5 ist der Rest des Diamins. Der Rest kann linear oder verzweigt sein oder Aromate enthalten oder daraus bestehen.

In einer Ausführung enthält R4 und/oder R5 beispielhaft mindestens eine hydrophile Einheit gemäß Formel I oder besteht daraus.

In einer Alternative ist das erfindungsgemäße Copolymer mithin das Kondensationsprodukt aus Dicarbonsäure der Formel III ― 1a (identisch mit II-1a):

HOOC-R4-COOH

mit einem Polyethylenglycoldiamin,
der Formel III-1b: H2N-CH2-CH2-O-(EO-)(I-2)-CH2-CH2-NH2.

In dieser Alternative ist R4 ein Alkyl aus 2-12 Methylengruppen, bevorzugt 4-8, besonders bevorzugt 4-6.

In einer weiteren Alternative ist das erfindungsgemäße Copolymer das Kondensationsprodukt einer Polyethylenglycoldicarbonsäure der Formel III-2a (identisch mit II-2a): HOOC-CH2-(EO-)(I-2)-CH2-COOH mit einem Diamin der Formel III-2b:

H2N-(CH2)m1-NH2,

und m1 = 2 - 20, bevorzugt 2-10 oder 4-12, besonders bevorzugt 2-8 oder 4-6.

In einer weiteren Alternative enthält das Copolymer mindestens zwei, bevorzugt vier Urethanfunktionen in der Hauptkette. Eine Urethanfunktion im Sinne der Erfindung ist eine funktionelle Gruppe, also eine Urethangruppe der Formel R6-NH-CO-O-R7, d.h. das Additionsprodukt eines Isocyanats mit einem Alkohol. Mithin sind die Monomere in der Hauptkette in dieser Alternative durch Urethanfunktionen miteinander verknüpft.

Das erfindungsgemäße Copolymer mit Urethanfunktion wird durch die allgemeine Formel IV dargestellt: -(R6-NRb-CO-O-R7-O-CO-NRb-)o;
o = 5 ― 500, bevorzugt 5-100 wobei die angegeben Mw Werte nicht überschritten werden und
R6 ist der Rest des Diisocyanats und R7 ist der Rest des Diols. Der Rest kann linear oder verzweigt sein oder Aromate enthalten oder daraus bestehen.

In einer Ausführung enthält R6 und/oder R7 mindestens eine hydrophile Einheit gemäß Formel I oder besteht daraus.

In einer Alternative ist das erfindungsgemäße Copolymer mithin das Additionsprodukt aus Diisocyanat der Formel IV ― 1a: O=C=N-R6-N=C=O
mit einem Polyalkoxydiol, bevorzugt Polyethylenglycol, der Formel IV-1b (identisch mit Formel I): HO-(EO-)I-H, wie oben beschrieben.

Bevorzugt wird PEG eingesetzt mit einem Molekulargewicht von 150-1000 g/mol, bevorzugt ca. 150 bis 600 g/mol, insbesondere 200 bis 450 oder 200 bis 300 g/mol. In dieser Alternative ist R6 ein Alkyl aus 2-10 Methylengruppen, bevorzugt 4-8, besonders bevorzugt 4-6.

In einer weiteren Alternative ist das erfindungsgemäße Copolymer das Additionsprodukt eines Polyethylenglycoldiisocyanats der Formel IV-2a:

O=C=N-(CH2)n4-(EO-)I-(CH2)n5-N=C=O

mit einem Diol der Formel IV-2b (identisch mit II-2b): HO-(CH2)n3-OH,
mit
n4=n5 = 2 ― 20, bevorzugt 2-10, besonders bevorzugt 2-8, insbesondere 2;
n3 = 2 - 20, bevorzugt 2-10, besonders bevorzugt 2-8 .

Eine weitere Ausführung der vorliegenden Erfindung betrifft das erfindungsgemäße Copolymer, welches ein Kondensationsprodukt aus Diolen ist und mithin mindestens 2, bevorzugt mindestens 4 Ether-Funktionen und/oder Derivate davon in der Hauptkette aufweist.

Im Sinne der Erfindung ist eine Ether-Funktion eine funktionelle Gruppe, also eine Ether-Gruppe der allgemeinen Formel (V) R8-O-R9. Mithin sind die Monomere der erfindungsgemäßen Copolymere über Ether-Gruppen in der Hauptkette miteinander verknüpft.

Zur Herstellung der erfindungsgemäßen Copolymere mit Ether-Funktionen werden als Ausgangsstoffe Diole eingesetzt, die ihrerseits bevorzugt Polyether sind, ausgewählt aus der Gruppe enthaltend oder bestehend aus Polyethylenglycol, Polypropylenglycol, Polybutylenglycol, Polytetramethylenglycol, Polyalkandiol und Polyether aufgebaut aus Alkoxyeinheiten mit 5 und mehr Kohlenstoffatomen. Bevorzugt werden die erfindungsgemäßen Copolymere aus hydrophilen Ausgangsstoffen wie Polyethylenglycol sowie hydrophoben Diolen wie z.B. Polypropylenglycol, Polybutylenglycol, Polytetramethylenglycol, Polyalkandiol und Polyether aufgebaut aus Alkoxyeinheiten mit 5 und mehr Kohlenstoffatomen eingesetzt.

In einer Ausführung enthält R8 und/oder R9 mindestens eine hydrophile Einheit gemäß Formel I oder besteht daraus. R8 und/oder R9 können linear oder verzweigt sein oder Aromate enthalten oder daraus bestehen.

In bevorzugten Ausführungen sind die Copolymere Polyether oder Polyester, besonders Polyester, wie beschrieben. Die funktionellen Endgruppen können Wirkstoffe binden.

Weiter bevorzugte Ausführungen sind:

In einer Ausführung dienen als Ausgangsstoffe für die hydrophilen Einheiten bevorzugt Polyethylenglycol oder Derivate des Polyethylenglycols, insbesondere ausgewählt aus der Gruppe enthaltend oder bestehend aus:
Polyethylenglycol (HO-(CH2—CH2-O)I-H), Polyethylenglycol-bis(carboxymethyl)ether (HOOC-CH2-O-(CH2-CH2-O)(I-2)CH2-COOH), Polyethylenglycoldiethylamin (H2N-(CH2-CH2-O-)(I-2)CH2-CH2-NH2) und Polyethylenglycol-bis(isocyanatomethyl)ether (OCN-CH2-O-(CH2-CH2-O-)(I-2)CH2-NCO).

In einer weiteren Ausführung dienen als Ausgangsstoffe für die hydrophoben Einheiten Kohlenwasserstoffreste, die zwei terminale funktionelle Gruppen tragen, bevorzugt ausgewählt aus der Gruppe:
Alkandiol (HO-(CH2)p-OH), Alkandicarbonsäure (HOOC-(CH2)(p-2)-COOH),
Alkandiamin (H2N-(CH2)(p-2)-NH2) und Alkandiisocanat (OCN-(CH2)(p-2)-NCO), mit p= 1-20, bevorzugt 1-12.

Der Kohlenwasserstoffrest (CH2)p kann linear sein oder auch verzweigt. Außerdem kann der Kohlenwasserstoffrest funktionelle Gruppen wie oben beschrieben, also komplementäre funktionelle Gruppe A oder B beinhalten.

In einer weiteren Ausführung sind die erfindungsgemäßen Copolymere das Reaktionsprodukt einer sog. Michael-Addition. Dabei werden hydrophobe und hydrophile Einheiten über Kohlenstoff-Kohlenstoffeinfachbindungen, Kohlenstoff-Schwefel, Kohlenstoff-Sauerstoff oder Kohlenstoff-Stickstoffbindungen verknüpft. Bei der Michael-Addition handelt es sich um die Addition eines sog. Michael-Donator an eine Alpha-Beta-ungesättigte Carbonyl-Verbindung, die als Michael-Akzeptor bezeichnet wird. Michael-Akzeptor-Akzeptoren sind z.B. Alpha, Beta-ungesättigte Aldehyde, Ketone-Ester oder Carbonsäureamide oder Alpha, Beta-ungesättigte Nitrile. Als Michael-Donator werden nukleophile und bevorzugt nach dem HSAB-Konzept relativ weiche Verbindungen eingesetzt, wie z.B. Verbindungen, die durch Zugabe einer Base in Alphastellung Anionen, also deprotonierte Carbonylverbindungen, bilden. Als solche Nukleophile können Amine, Thiole, Phenolationen oder Cyanide eingesetzt werden. Für die erfindungsgemäßen Copolymere werden als Edukte Verbindungen eingesetzt, die jeweils zwei funktionelle Gruppen für eine Michael-Addition aufweisen, also entweder zwei Michael-Akzeptoren, zwei Michael-Donatoren oder ein Michael-Akzeptor und ein Michael-Donator. In den Edukten oder mindestens einem Edukt kann zwischen den beiden funktionellen Gruppen (Michael-Akzeptor, Michael-Donator) eine EO-(I) Kette vorliegen. Als Michaelakzeptoren eignen sich insbesondere Polyethylenglykoldimethacrylate oder Polyethylenglykoldiacrylate (an beiden Kettenenden mit Methacrylsäure oder Acrylsäure verestertes Polyethylenglycol). Als Michaeldonator eignen sich insbesondere Alkandithiole wie 1,4-Butandithiol oder 1,6-Hexandithiol.

Als Ausgangsstoffe für die hydrophoben Einheiten können auch hydrophobe

Polymere verwendet werden, sofern sie funktionelle Endgruppen tragen. Besonderes eigenen sich Polyether wie Polypropylenglycol, Polybutylenglycol oder Polytetrahydrofuran. Die Endgruppen können Alkohole, Amine oder auch andere funktionelle Gruppen sein.

Aus den Ausgangsstoffen der hydrophilen oder hydrophoben Einheiten werden durch geeignete Polymerisationsverfahren die Polymere bestehen aus alternierend hydrophilen und hydrophoben Einheiten hergestellt. Geeignete Polymerisationsverfahren sind z.B. Polykondensation oder Polyaddition. Dadurch werden z.B. Polyester, Polyamide, Polyether oder Polyurethane erhalten. Beispielsweise führt die Polymerisation eines Polyethylenglycols mit einer Dicarbonsäure zu einem Polyester der Struktur -(OC-(CH2)p-2-CO-O(CH2-CH2-O)I)x-. Alternativ kann der Polyester auch durch Umsetzung eines Polyethylenglycol-bis(carboxymethyl)ethers mit einem Alkandiol erhalten werden.

Durch Umsetzung eines Polyethylenglycol-bis(carboxymethyl)ethers mit einem Alkandiamin wird ein Polyamid erhalten, welches folgende Struktur hat; -(OC-CH2-O-(CH2-CH2-O-)I-CH2-CO-(NH)-(CH2)p2-(NH))x-, wobei x die Zahl der sich wiederholenden Einheiten definiert und sich aus der oben beschriebenen Molmasse ergibt.

Alternativ zu den oben genannten Verbindungen können auch andere eingesetzt werden, um die Reaktivität zu erhöhen. So kann beispielsweise eine Carbonsäuregruppe in ein Säurechlorid umgesetzt werden, um daraus einen Polyester oder ein Polyamid herzustellen. Zur Herstellung von Polyethern aus beispielsweise einem Polyethylenglycol und einem Diol kann eine der beiden Komponenten intermediär zum entsprechenden Ditosylat umgesetzt werden, um dann den Polyether herzustellen, der folgende Struktur besitzt: -(O-(CH2-CH2-O)I-(CH2)p2)x-.

Statt primären Aminen können auch sekundäre Amine eingesetzt werden. Bevorzugt werden dabei methylierte Amine, die beispielsweise die Struktur H(CH3)N-(CH2)(p-2)-N(CH3)H besitzen.

Die Endgruppen der Polymerketten ergeben sich aus den eingesetzten Ausgangsstoffen. In einem Polyester treten beispielsweise Carbonsäure- und Alkoholgruppen auf. Die Endgruppen haben keinen entscheidenden Einfluss auf das Translokationsverhalten der Polymere. Die Endgruppen können auch durch Postpolymerisationsreaktionen verändert werden, um z.B. Wirkstoffmoleküle zu binden.

Statt linearer Ketten sind auch verzweigte Strukturen möglich, die beispielsweise durch Verwendung von Gemischen aus difunktionellen und trifunktionellen Ausgangsstoffen erhalten werden können. Der Anteil mehr als difunktionellem Ausgangsstoff muss aber so gering sein, dass dabei immer noch ein löslicher Polymer erhalten wird.

Die Ausgangsstoffe der hydrophilen und hydrophoben Einheiten können monodispers sein. Sie können aber auch eine Molekulargewichtsverteilung aufweisen. Die gilt insbesondere für polymere Ausgangsstoffe. Auch niedermolekulare Ausgangsstoffe, wie Dicarbonsäuren können als Gemische verschiedener Kettenlängen verwendet werden.

Die vorliegende Offenbarung betrifft auch Verfahren zur Herstellung der oben beschriebenen Copolymere.

Vorteilteilhaft an dem Verfahren ist die einfache Darstellung der Copolymere, die in eine Alternative ohne weitere Aufarbeitung sofort einsetzbar sind.

In einer anderen Alternative kann eine Aufarbeitung erfolgen, insbesondere Aufreinigung der Polymere.

Hier können durch unterschiedliche Löslichkeit der Polymere bestimmte Fraktionen mit engerer Molmassen-Verteilung getrennt und aufgereinigt werden.

Zur Trennung werden alle Polymere bevorzugt in Wasser oder anderen Lösungsmittel gelöst und durch Zugabe von Nicht-Lösungsmitteln werden die entsprechenden Copolymerfraktionen gemäß ihrer Löslichkeit ausgefällt. Dieses Trennverfahren ist temperatur-abhängig.

Die Trennung kann auch durch eine Temperaturänderung erfolgen.

Dadurch werden nieder- und hochmolekulare Fraktionen getrennt. Dies entspricht der Fraktionierung, siehe Beispiele.

Die Bestimmung der Molekulargewichte sowohl Mw als auch Mn der Copolymere kann erfindungsgemäß mittels Gel-Permationschromatographie, gegebenenfalls gekoppelt mit Online Lichtstreudedektion (GPC - LS) oder NMR mittels Endgruppenbestimmung erfolgen.

Des Weiteren erfolgt im Sinne der Erfindung die Ermittlung der Anteile an OH- sowie COOH- Endgruppen über 1 H-MNR zur Berechnung des Molekulargewichts (Mn).

Die vorliegende Erfindung betrifft auch ein Copolymer, wie oben beschrieben, zur Verwendung als Träger für Wirkstoffe, also Mittel mit biologischer Wirkung. Das Copolymer dient als Träger für Wirkstoffe, Mittel mit biologischer Wirkung, da es diesen Stoffen eine Translokation durch eine Lipid-Bilayer-Membran, bevorzugt Zellmembran ermöglicht, also die Translokation der Stoffe bewirkt und erst ermöglicht.

Im Sinne der Erfindung sind Wirkstoffe oder Mittel mit biologischer Wirkung gleich zu verwenden. Wirkstoffe sind in einer Ausführung Arzneimittel, wie sie im Arzneimittel-Gesetz § 2 (1) und § 2 (2) sowie § 4 (19) Stand 04.04.2016 definiert sind.

In einer Alternative sind unter Wirkstoffe alle Stoffe mit einer pharmazeutischen und/oder biologischen Wirkung zu verstehen. Wirkstoffe sind somit Verbindungen, ausgewählt aus der Gruppe bestehend aus pharmazeutisch aktiven Verbindungen, therapeutisch wirksamen Verbindungen und biologisch aktiven Verbindungen, kosmetisch aktiven Verbindungen, Stoffe, die biochemische und/oder physiologische Prozesse in einen Organismus und/oder quantitativ beeinflussen, d.h. fördern, überhaupt erst ermöglichen oder hemmen.

Wirkstoffe entfalten ferner in kleinen Mengen eine große pharmazeutische, chemische, biologische oder physiologische Wirkung.

In einer Ausführung sind Wirkstoffe auch Farbstoffe, Fluoreszenzmarker, radioaktive Marker.

In einer weiteren Ausführung sind Wirkstoffe bzw. Mittel mit biologischer Wirkung auch Biozide.

Die oben beschriebenen Copolymere weisen in einer Ausführung neben den oben beschriebenen Esterfunktionen und OH- sowie COOH-Endgruppen keine weiteren funktionellen Gruppen auf. Entsprechend weisen die oben beschriebenen Copolymere als Kondensationsprodukt von Dicarbonsäuren mit Aminen und Additionsprodukt von Isocyanaten mit Diolen neben den Amidfunktionen bzw. Urethanfunktionen und den COOH- und NH2-Endgruppen bzw. OH-Endgruppen in einer Ausführung keine weiteren funktionellen Gruppen auf.

In einer weiteren Alternative besitzen die oben beschrieben Copolymere funktionelle Gruppen. Funktionelle Gruppe sind wie oben beschriebene Reste bzw. Moleküle, die kovalent an die Copolymere gebunden sind. Funktionelle Gruppen können eingeführt werden, um die chemischen und physikalischen Eigenschaften der Copolymere zu verändern oder um Wirkstoffe, Mittel mit biologischer Wirkung, daran zu binden.

Die Erfindung betrifft mithin die oben beschriebene Copolymere verbunden, verknüpft mit dem Wirkstoff, wobei die Verknüpfung eine kovalente Bindung ist.

Die vorliegende Erfindung betrifft in einer weiteren Ausführung die oben beschriebenen Copolymere, verknüpft mit einem Wirkstoff, wobei die Verknüpfung keine kovalente Bindung ist.

Die Verknüpfung erfolgt hier über Wasserstoffbrücken, hydrophile, hydrophobe, elektrostatische Wechselwirkung bzw. Bindung und/oder sterische Immobilisierung. Eine nicht kovalente Verknüpfung bietet gegenüber der kovalenten Verknüpfung eine einfachere Freisetzung der Wirkstoffe, da keine chemische Bindung gespaltet werden muss, sondern Änderungen der physikalischen Umgebung schon zu einer Freisetzung führen können.

In einer Alternative können Wirkstoffe zur nicht-kovalenten Verknüpfung in die Überstruktur der oben beschriebenen Copolymere eingeschlossen werden.

Gegenstand der vorliegenden Erfindung ist auch das oben beschriebene Copolymer, verknüpft mit einem Wirkstoff, bzw. einem Mittel mit biologischer Wirkung, zur Verwendung als Medikament.

Weiterer Gegenstand der Offenbarung ist die Verwendung des oben beschriebenen Copolymers als Träger für Wirkstoffe und Mittel mit biologischer Wirkung für den Transport durch eine Lipid-Bilayer-Membran, insbesondere Zellmembran. Gegenstand ist auch die Verwendung des oben beschriebenen Copolymers, verknüpft mit einem Wirkstoff und/oder Mittel mit biologischer Wirkung als Medikament. Die Formulierung erfolgt durch den Fachmann bekannte Verfahren, wie sie z.B. in Arzneiformellehre, 4. Aufl., von Ursula Schöffling 2003 beschrieben werden.

In einer Ausführung werden die oben beschriebenen Copolymere jedoch ex-vivo verwendet und eingesetzt, um z.B. als Träger wie oben beschrieben, die Translokation unterschiedlicher Wirkstoffe in vitro zu testen. Hierzu erfolgen Tests, durchgeführt wie sie in den Beispielen beschrieben werden. Insbesondere ist hierzu eine künstliche Lipid-Bilayer-Membran geeignet.

Die Copolymere und Wirkstoffe werden erfindungsgemäß aufeinander abgestimmt. Liegt zum Beispiel ein hydrophober Wirkstoff mit hohem Molekulargewicht vor, wird er an Copolymer mit höherem Molekulargewicht und/oder mit höherem LCST-Wert verbunden. Somit wird ein gesamt stärker hydrophiler Charakter des mit dem Wirkstoff verknüpften Copolymers erreicht.

In einer Ausführung werden unterschiedliche Copolymere - in Bezug auf die chemische Zusammensetzung der Ausgangsstoffe oder bezüglich des Molekulargewichts - mit gegebenenfalls unterschiedlichen Wirkstoffen eingesetzt, um so eine Translokation eines Wirkstoffs über einen längeren Zeitraum zu gewährleisten oder um eine genaue Abfolge von unterschiedlichen Wirkstoffen zu gewährleisten.

Gegenstand der vorliegenden Offenbarung ist auch Kit, enthaltend die oben beschriebenen Copolymere. In einem solchen Kit liegen in einer Alternativen die oben beschriebenen Copolymere in Lösung vor. In einer anderen Alternative liegen sie als Pulver vor. Des Weiteren kann ein Kit z.B. Behälter mit Puffern oder Lösungen enthalten. Alle Komponenten können in demselben Behälter oder getrennt voneinander verpackt sein. Ferner kann auch eine Anweisung für den Gebrauch des Kits vorliegen. Die Copolymere können beispielsweise in einer Induktionsflasche mit Stopfen oder Septum vorliegen. Daneben kann das Kit auch eine Einmal-Spritze zur Entnahme der Proben enthalten.

Die oben beschriebenen Copolymere sowie das entsprechende Herstellungsverfahren bieten die Möglichkeit, genau abgestimmte Copolymere herzustellen, insbesondere bezüglich ihrer hydrophoben und hydrophilen Eigenschaften sowie LCST-Werte. Da beispielsweise die Amid- und/oder Urethanfunktionen polarer als die Esterfunktionen sind, können hydrophobe Elemente, wie z.B. CH2-Ketten zwischen diesen Funktionen eingebaut werden, um einen höheren hydrophoben Anteil zu erreichen.

### Beispiele:

### 1. Herstellung der erfindungsgemäßen Polymere

P(C4EG6):16,81 g Bernsteinsäureanhydrid (168 mmol) wurde in einem Glaskolben mit 49,97 g Polyethylenglykol 300 (Mn = 298 g/mol, 168 mmol) und 53 mg Toluolsulfonsäure-Monohydrat gemischt und unter Rühren für einen Tag unter Argon auf 120°C erhitzt. Danach wurde der Druck auf 100 mbar abgesenkt und innerhalb von 2 Stunden wurde auf 160 °C erwärmt. Bei dieser Temperatur wurde der Druck weiter auf 20 mbar gesenkt. Nach 16 Stunden wurde der Druck weiter auf ca. 0,1 mbar reduziert und weitere 4 Tage geheizt. Das so erhaltene Produkt wurde ohne weitere Aufarbeitung weiter verwendet.

P(C5EG6): 12,91 g Glutarsäure (97,5 mmol) wurde in einem Glaskolben mit 29,01 g Polyethylenglykol 300 (Mn = 298 g/mol, 97,4 mmol) und 37 mg Toluolsulfonsäure-Monohydrat gemischt und unter Vakuumbedingungen (ca. 0,1 mbar) erhitzt und gerührt. Es wurde jeweils eine Stunde auf 80°C, 100°C und 120°C erhitzt. Anschließend wurde die Temperatur für 3 Stunden auf 140°C erhöht und abschließend 62 Stunden bei 160°C geheizt. Das so erhaltene Produkt wurde ohne weitere Aufarbeitung weiter verwendet.

P(C6EG6): 24,24 g Adipinsäure (166 mmol) wurde in einem Glaskolben mit 49,54 g Polyethylenglykol 300 (Mn = 298 g/mol, 166 mmol) und 58 mg Toluolsulfonsäure-Monohydrat gemischt und bei 100 mbar erhitzt und gerührt. Es wurde jeweils eine Stunde auf 120°C, 140°C und 160°C erhitzt. Anschließend der Druck auf 50 mbar abgesenkt und bei 160°C 16 Stunden weiter geheizt. Bei dieser Temperatur wurde der Druck innerhalb eines Tages auf ca. 0,1 mbar reduziert und unter diesen Bedingungen das Gemisch 4 weitere Tage belassen. Das so erhaltene Produkt wurde ohne weitere Aufarbeitung weiter verwendet.

P(C4EG4):32,58 g Bernsteinsäureanhydrid (326 mmol) wurde in einem Glaskolben mit 63,21 g Tetraethylenglykol (325 mmol), 351 mg Toluolsulfonsäure-Monohydrat und 117 g Toluol gemischt und unter Rühren am Rückfluss unter Zwischenschalten eines Wasserabscheiders zum Sieden erhitzt. Nach 2 Tagen wurde der Druck auf ca. 0,1 mbar gesenkt und das Gemisch bei 120 °C für einen weiteren Tag geheizt. Das so erhaltene Produkt wurde ohne weitere Aufarbeitung weiter verwendet.

P(C8EG13): 15,53 g Suberinsäure (89,2 mmol) wurde in einem Glaskolben mit 53,40 g Polyethylenglykol 600 (Mn = 599 g/mol, 89,1 mmol) und 70 mg Toluolsulfonsäure-Monohydrat gemischt und bei 100 mbar erhitzt und gerührt. Es wurde jeweils eine Stunde auf 120°C, 140°C und 160°C erhitzt. Anschließend der Druck auf 10 mbar abgesenkt und bei 160°C 16 Stunden weiter geheizt. Bei dieser Temperatur wurde der Druck innerhalb eines Tages auf ca. 0,1 mbar reduziert und unter diesen Bedingungen das Gemisch 2 weitere Tage belassen. Das so erhaltene Produkt wurde ohne weitere Aufarbeitung weiter verwendet.

Vergleich: P(C10EG22): 15,16 g Sebacinsäure (75,0 mmol) wurde in einem Glaskolben mit 73,95 g Polyethylenglykol 1000 (Mn = 986 g/mol, 75,0 mmol) 85 mg Toluolsulfonsäure-Monohydrat und 112 g Toluol gemischt und unter Rühren am Rückfluss unter Zwischenschalten eines Wasserabscheiders zum Sieden erhitzt. Nach einem Tag wurde der Druck auf ca. 0,1 mbar gesenkt und das Gemisch bei 140 °C für 5 weitere Tage geheizt. Das so erhaltene Produkt wurde ohne weitere Aufarbeitung weiter verwendet.

P(EG4PG3): 33,16 g Poly(ethyleneglycol) bis(carboxymethyl) ether (Mn = 217 g/mol, 153 mmol) wurde in einem Glaskolben mit 29,38 g Tripropylenglykol (153 mmol) und 110 mg Toluolsulfonsäure-Monohydrat gemischt und bei 100 mbar für eine halbe Stunde auf 120 °C erhitzt und gerührt. Danach wurde bei 140 °C innerhalb von 3,5 Stunden der Druck auf 5 mbar reduziert. Nach weiteren 18 Stunden wurde die Temperatur auf 160 °C erhöht und dann noch 2 Tage bei 5 mbar und ein Tag bei ca. 0,1 mbar gerührt. Das so erhaltene Produkt wurde ohne weitere Aufarbeitung weiter verwendet.

Fraktionierung von P(C5EG6): 7,35 g P(C5EG6) wurden in 500 mL Toluol gelöst und unter Rühren bei Raumtemperatur langsam 1,4 L Ethanol zugegeben bis das Gemisch deutlich trüb war. Durch leichtes Erwärmen wurde eine klare Lösung erhalten, die über mehrere Stunden wieder auf Raumtemperatur abgekühlt wurde. Nach Absetzen der sich dabei bildenden unteren Phase wurde das überstehende, klare Gemisch abdekantiert, mit weiterem Ethanol versetzt, erwärmt bis die Mischung klar wurde, langsam auf Raumtemperatur abgekühlt und die überstehende klare Flüssigkeit von der zweiten unteren Phase abgetrennt. Dieser Prozess wurde noch zweimal wiederholt. Insgesamt wurden 3,3 L Ethanol zugegeben. Die 4 unteren Phasen wurden getrocknet, mit Ethanol gewaschen und abschließend getrocknet, um die Fraktionen P(C5EG6)-F1 bis P(C5EG6)-F4 zu erhalten. Das noch im Toluol/Ethanol-Gemisch gelöste Polymer wurde durch Abdestillieren der Lösemittel isoliert und bei 70 °C in 1 L Ethanol gelöst. Durch schrittweises Abkühlen wurden zwischen 39 °C und -15 °C 4 weitere Fraktionen erhalten, die getrocknet wurden (Fraktionen P(C5EG6)-F5 bis P(C5EG6)-F8). Jede der Fraktionen umfasste zwischen etwa 0,5 g bis 1 g Polymer.

### Beispiel 2: Charakterisierung der Polymere.

Absolute Molekulargewichte (Zahlenmittleres Molekulargewicht Mn und Gewichtsmittleres Molekulargewicht Mw vorher) sowie die Polydispersität Mw/Mn der Polymerprodukte wurden mittels Gelpermeationschromatographie, gekoppelt mit Online-Lichtstreudetektion (GPC-LS) in THF als Laufmittel ermittelt. Mn sowie die Anteile an OH- sowie COOH-Endgruppen (% OH, % COOH) wurden über 1H-NMR in deuteriertem Pyridin ermittelt. Die die Polydispersität Mw/Mn wurde zusätzlich über GPC mit PEO Kalibrierung (GPC-PEO) ermittelt. In diesem Fall wurde ein Laufmittelgemisch aus 84 Gew.-% THF, 15 Gew.-% Dimethylacetamid und 1 Gew.-% Essigsäure verwendet Die Ergebnisse sind in Tabelle 1 zusammengefasst.

Nicht fraktionierte Copolymere haben eine breite Molekulargewichts-Verteilung. Da mittels GPC-LS niedermolekulare Anteile nicht vollständig erfasst werden, liegen diese Werte etwas unter den mittels GPC-PEO bestimmten Werten.

**Tabelle 1:**

| | | GPC-LS | | NMR | | | GPC-PEO | Translokation |
|---|---|---|---|---|---|---|---|---|
| | LCST | Mn | Mw/M n | % OH | % COOH | Mn | Mw/M n | |
| P(C5EG6) | 42°C | 11.700 | 1,51 | 34 | 66 | 12.000 | 2,46 | + |
| P(C4EG6) | 64 °C | 6.590 | 1,58 | 62 | 38 | 5.340 | 1,97 | + |
| P(C6EG6) | 28 °C | 8.700 | 1,59 | 53 | 47 | 7.750 | 2,20 | + |
| P(C5EG14) | 83 °C | 6.390 | 1,52 | 67 | 33 | 5.210 | 2,23 | + |
| P(C8EG13) | 47 °C | 3.780 | 1,52 | 58 | 42 | 3.070 | 2,00 | + |
| P(C4EG4) | 29°C | 5.160 | 1,38 | 45 | 55 | 8.230 | 2,58 | + |
| P(C10EG22) (Vergleich) | 35 °C | 10.100 | 1,49 | 59 | 41 | 9.750 | 1,65 | - |
| P(EG4PG3) | 21 °C | 20.100 | 1,63 | | | | | |
| P(C5EG6)-F1 | | 34.400 | 1,16 | | | | | + |
| P(C5EG6)-F2 | | 25.100 | 1,08 | | | | | + |
| P(C5EG6)-F3 | | 16.600 | 1,07 | | | | | + |
| P(C5EG6)-F4 | | 12.900 | 1,05 | | | | | |
| P(C5EG6)-F5 | | 9.300 | 1,07 | 33 | 67 | 11.600 | | + |
| P(C5EG6)-F6 | | 6.400 | 1,07 | | | | | + |
| P(C5EG6)-F7 | | 4.200 | 1,12 | | | | | + |
| P(C5EG6)-F8 | | 2.290 | 1,15 | | | | | + |

Gemäß oben verwendeter Nomenklatur steht P für ein Polymer welches ein Copolymer-Polyester ist. Die Säure-Einheit wird an erster Stelle definiert; diese enthält zum Beispiel eine Kette aus z4 C-Atome für C4, 5 C-Atome für C5 (C5 ist HOOC-(CH2)3-COOH) usw.. Die Alkohol-Einheit wird an zweiter Stelle definiert und zum Beispiel eine Kette aus 6 kondensierten Ethylenglykol-Untereinheiten für EG6 und eine Kette aus 14 kondensierten Ethylenglykol-Untereinheiten für EG14 enthält usw.. Steht an erster Stelle für die Dicarbonsäure zum Beispiel EG4, handelt es sich bei dem Ausgangsstoff um eine Polyethylenglycoldicarbonsäure auf Basis eines Ethylenglycols mit 4 ca. EO-Einheiten, wobei die terminalen C-Atome in Carboxylgruppen vorliegen.

### Beispiel 3:

### Ermittlung der Membrangängigkeit der Polymere

Zur Bestimmung der Membrangängigkeit der erfindungsgemäßen Polymere, also der Translokalation durch eine Membran wurde eine Variante des sogenannten Droplet Interface Bilayer DIB verfahrens verwendet gemäß Bayley von 2008 in Journal Anal Chem. Gemäß diesem Verfahren wurde eine freistehende Lipid-Bilayer-Membran in einem Mikrofluidchip erzeugt.

Die Mikrokanäle der Chips waren so angeordnet, dass sie sich überkreuzen. Die Mikrokanäle wurden zunächst mit einer Phosporlipidmischung (DOPC, 1,2-Dioleoyl-sn-glycero-3-phosphocholine) gelöst in Squalen befüllt.

An zwei gegenüberliegenden Enden wurde eine wässrige Phase, enthaltend 100 mM NaCl in zwei Enden der Kanäle injiziert mittels einer Volumen-kontrollierten Pumpe für Injektionsspritzen.

Die wässrige Phase aus den Spritzen bildete zwei sogenannte Finger bzw. Blasen, die sich von den Injektionsspritzen weg vergrößern. Nach einigen Sekunden bildete sich an der Phasengrenze zwischen Wasser- und Ölphase jeweils ein Lipid Monolayer der sich über den ganzen Finger erstreckte. Sobald sich die zwei Finger mit dem Lipid Monolayer berührten, wurde aus diesen zwei Lipid-monolayer ein Lipid-Bilayer gebildet. Dieser Prozess erfolgt nicht unmittelbar, sondern auf Basis des Rückzugs bzw. des Ableitens des Öls, hier des Squalen, zwischen den beiden Lipid-Monolayer während eines sogenannten "Zipping-Prozesses". Nach seiner Bildung war der Bilayer stabil und konnte gleichzeitig durch optische Mikroskopie als auch durch elektrophysiologische Experimente analysiert werden.

Die erfindungsgemäßen Polymere wurden in einer der Spritzen in der wässrigen Phase dispergiert beziehungsweise gelöst, so dass in einem der Finger die erfindungsgemäßen Polymere anwesend waren, während in dem anderen Finger nicht. Dieser enthielt nur die wässrige 100 mM NACL-Lösung in reinem Wasser (z.B. destilliertes Wasser oder entmineralisiertes).

Somit waren lediglich auf einer Seite der gebildeten Phospholipid-Bilayer-Membran die erfindungsgemäßen Polymere anwesend. Nach 24 Stunden wurden Proben (ca. 100 bis 200 µl mittels eine Mikropipette) aus der wässrigen Phase entnommen, die ursprünglich die erfindungsgemäßen Polymere nicht enthielt und chemisch analysiert.

### Erst wurden Proben aus mehreren Mikrofluidchips bzw. mehreren Experimenten entnommen bis ca. eine Menge von einem Milliliter vorlag.

Die chemische Analyse der wässrigen Proben wurde wie folgt durchgeführt:
Das Wasser wurde durch Gefriertrocknung entfernt. Die erhaltenen Rückstände wurden mittels GPC und 1H-NMR charakterisiert. Als Vergleich bzw. Kontrolle wurde die ursprüngliche Lösung enthaltend die erfindungsgemäße Polymere, die auf eine Seite injiziert wurde, ebenfalls auf diese Art chemisch analysiert.

Für die erfindungsgemäßen Copolymere: P(C5EG6), P(C4EG6), P(C6EG6)P und (C8EG13) waren GPC-Chromatogramme und NMR-Spektren der Kontrolle mit jenen der entnommenen Proben identisch.

Bestimmung der Permeationskinetik mittels Osmometrie:
Wie oben beschrieben, wurde der Lipid Bilayer durch Kontakt zweier Wassertropfen in einer Ölphase gebildet. Die beiden sog. Wasser-Finger wurden mittels eines druckgesteuerten Systems in Mikrokanäle injiziert. Das druckgesteuerte System bestand aus einem hydrostatischen Reservoir, das unmittelbar mit dem Mikrokanal verbunden war. Die zwei Wasserfinger wurden mithin von beiden Seiten des Mikrokanalsystems aufeinander zugeschoben, bis sie miteinander in Kontakt traten und so den Lipid Bilayer bildeten. Dabei wurde die kontinuierliche Ölphase, die ursprünglich in den Mikrokanälen war, verdrängt. Nach Kontakt der beiden Tropfen, bzw. in diesem Fall der Wasser-Finger und der darauf basierenden Bildung des Lipid-Bilayers, stellte sich ein mechanisches Gleichgewicht ein, da der auf die beiden Wasser-Finger wirkende Druck ausgeglichen wurde. Der hydrostatische Druck, der auf jeden der beiden Wasser-Finger wirkte, ist präzise durch die Höhe des Wasserreservoirs eingestellt (Ph = p × g × h). einer der beiden Wasser-Tropfen (Wasser-Finger) war reine wässrige Pufferlösung, während der andere die erfindungsgemäßen Polymere in einer Konzentration von ca. 5 mg/ml in demselben Puffer enthielt. Aufgrund der Translokation durch den Lipid-Bilayer änderte sich die Polymerkonzentration in den beiden Wasser-Finger über die Zeit und dadurch brach das mechanische Gleichgewicht zusammen. Dies bewirkte seinerseits wieder eine sehr langsame Verschiebung des Lipid-Bilayers innerhalb des Mikrokanals. Die Lage des Lipid-Bilayers wurde mittels Mikroskop genau festgehalten. Die Position des Lipid-Bilayers wurde neu eingestellt durch Veränderung des Drucks auf einen Wasser-Finger, im Einzelnen durch Veränderung der Höhe des Wasserreservoirs. Die Änderung des hydrostatischen Drucks, die erforderlich war, um das Gleichgewicht des Bilayers wiederherzustellen, drückte unmittelbar die Änderung im osmotischen Druck aus, die durch die Änderung der Polymerkonzentration verursacht wurde.

Als Referenzmaterial wurde Polyethylenglykol mit einem Molekulargewicht von Mn = 2,000 g/mol verwendet.

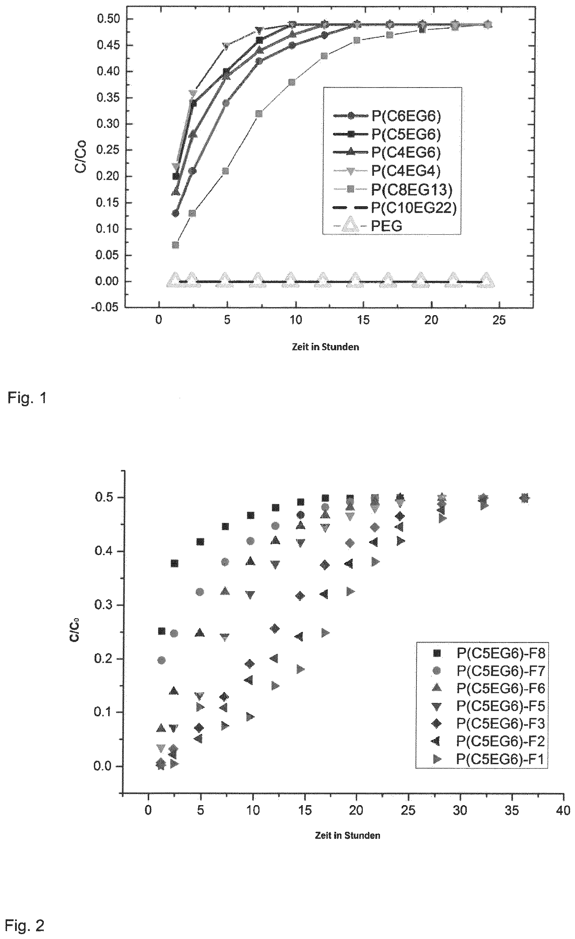

Fig. 1 und 2 zeigen die Translokationskinetik, die mittels Osmometrie gemessen wurde. Der Wert von C/Co von 0 zu Beginn der Messung bedeutet, dass sich alles Polymer auf einer Membranseite befindet. Ein Wert von 0,5 bedeutet, dass auf beiden Seiten der Membran die Polymerkonzentration identisch ist.

In Fig. 1 überlappen die Werte für PEG und P(C10EG22).

Daraus geht eindeutig hervor, dass eine Translokation der Copolymere durch die Bilayer-Membran stattgefunden hat.

## Patentansprüche

1. Copolymer kovalent oder physikalisch mit mindestens einem Wirkstoff verbunden enthaltend alternierende hydrophobe und hydrophile Einheiten,
**dadurch gekennzeichnet, dass**
das Copolymer eine untere kritische Lösungstemperatur (LCST-Wert) von 20°C bis 90°C aufweist, eine im Wesentlichen lineare Hauptkette enthält oder daraus besteht, und im Wesentlichen nicht-ionisch ist,
**dadurch gekennzeichnet, dass**
die hydrophile Einheit durch die Formel -(CH₂-CH₂-O-)I mit I = 2 bis 20 definiert ist, und
wobei das Copolymer durch eine zahlenmittlere Molmasse Mn von 2000 bis 50000 g/mol charakterisiert ist, und wobei der LCST-Wert ein Temperatur-Intervall um den bestimmten Wert von 30% oder 20% des jeweiligen Wertes betrifft.

2. Copolymer nach Anspruch 1, wobei das Copolymer durch eine zahlenmittlere Molmasse Mn von 2500 bis 25000 g/mol, charakterisiert ist.

3. Copolymer nach Anspruch 1 oder 2, wobei das Copolymer durch eine zahlenmittlere Molmasse Mn von 3000 bis 14000 g/mol charakterisiert ist.

4. Copolymer nach einem der vorangehenden Ansprüche mit einem LCST-Wert von 20°C bis 80°C, bevorzugt 20°C bis 70°C.

5. Copolymer nach einem der vorangehenden Ansprüche,
**dadurch gekennzeichnet, dass**
I = 3 bis 8, bevorzugt 4 bis 6 ist.

6. Copolymer nach einem der vorangehenden Ansprüche,
**dadurch gekennzeichnet, dass**
mindestens vier, bevorzugt mindestens sechs Ether-Funktionen in der Hauptkette vorliegen.

7. Copolymer nach einem der Ansprüche 1 bis 5,
**dadurch gekennzeichnet, dass**
mindestens zwei, bevorzugt mindestens vier Funktionen in der Hauptkette vorliegen, ausgewählt aus der Gruppe enthaltend oder bestehend aus Ester-, Amid- und/oder Urethan- Funktionen und/oder Kombinationen davon.

8. Copolymer nach einem der vorangehenden Ansprüche zur Verwendung als Träger für Wirkstoffe durch eine Doppellipidschicht.

9. Copolymer nach Anspruch 8,
**dadurch gekennzeichnet, dass**
die Doppellipidschicht Teil einer biologischen Membran, bevorzugt Zellmembran ist.

10. Copolymer nach einem der Ansprüche 6 bis 9 zur Verwendung als Medikament.

11. Verwendung eines Copolymers nach einem der Ansprüche 1 bis 9 zum Transport von Wirkstoffen durch eine Doppellipidschicht in vitro.

12. Verwendung nach Anspruch 11,
**dadurch gekennzeichnet, dass**
die Doppellipidschicht Teil einer biologischen Membran, bevorzugt Zellmembran ist.

13. Verfahren zum Transport eines Wirkstoffs durch eine Doppellipidschicht in vitro, bei welchem ein Copolymer nach einem der Ansprüche 1 bis 9 in einer wässrigen Lösung auf einer Seite der Doppellipidschicht bereitgestellt wird.

## Claims

1. Copolymer covalently or physically connected to at least one active ingredient wherein the copolymer contains alternating hydrophobic and hydrophilic units, **characterized in that** the copolymer has a lower critical solution temperature (LCST value) of from 20°C to 90°C, contains or consists of a substantially linear main chain, and is substantially non-ionic, **characterised in that** the hydrophilic unit is defined by the formula -(CH₂-CH₂-O-)I with I = 2 to 20, and wherein the copolymer is **characterised by** a number average molecular weight Mn of from 2000 to 50000 g/mol, and wherein the LCST value concerns a temperature interval around the determined value of 30% or 20% of the particular value.

2. Copolymer according to claim 1, wherein the copolymer is **characterised by** a number average molecular weight Mn of from 2500 to 25000 g/mol.

3. Copolymer according to claim 1 or 2, wherein the copolymer is **characterised by** a number average molecular weight Mn of from 3000 to 14000 g/mol.

4. Copolymer according to one of the preceding claims having an LCST value of from 20°C to 80°C, preferably from 20°C to 70°C.

5. Copolymer according to one of the preceding claims, **characterized in that** I = 3 to 8, preferably 4 to 6.

6. Copolymer according to one of the preceding claims, **characterized in that** at least four, preferably at least six ether functions are present in the main chain.

7. Copolymer according to one of claims 1 to 5, **characterized in that** at least two, preferably at least four, functions are present in the main chain, selected from the group comprising or consisting of ester, amide and/or urethane functions and/or combinations thereof.

8. Copolymer according to one of the preceding claims for use as a carrier for active ingredients through a lipid bilayer.

9. Copolymer according to claim 8,
**characterized in that** the lipid bilayer is part of a biological membrane, preferably cell membrane.

10. Copolymer according to one of claims 6 to 9 for use as a medicament.

11. Use of a copolymer according to one of claims 1 to 9 for transporting active ingredients through a lipid bilayer in vitro.

12. Use according to claim 11, **characterized in that** the lipid bilayer is part of a biological membrane, preferably cell membrane.

13. Method for transporting an active ingredient through a lipid bilayer in vitro, in which a copolymer according to one of claims 1 to 9 is provided in an aqueous solution on one side of the lipid bilayer.

## Revendications

1. Copolymère lié de manière covalente ou physique à au moins un principe actif contenant des motifs hydrophobes et hydrophiles alternés,
**caractérisé en ce que**
le copolymère présente une température de solution critique inférieure (valeur LCST) de 20°C à 90°C, contient ou consiste en une chaîne principale sensiblement linéaire, et est sensiblement non ionique,
**caractérisé en ce que**
le motif hydrophile est défini par la formule -(CH₂-CH₂-O-)I avec I = 2 à 20, et
le copolymère est **caractérisé par** une masse molaire moyenne en nombre Mn de 2000 à 50000 g/mol, la valeur LCST concernant un intervalle de température autour de la valeur déterminée de 30 % ou de 20 % de la valeur respective.

2. Copolymère selon la revendication 1,
dans lequel le copolymère est **caractérisé par** une masse molaire moyenne en nombre Mn de 2500 à 25000 g/mol.

3. Copolymère selon la revendication 1 ou 2,
dans lequel le copolymère est **caractérisé par** une masse molaire moyenne en nombre Mn de 3000 à 14000 g/mol.

4. Copolymère selon l'une des revendications précédentes, ayant une valeur LCST de 20 °C à 80 °C, de préférence de 20 °C à 70 °C.

5. Copolymère selon l'une des revendications précédentes,
**caractérisé en ce que**
I = 3 à 8, de préférence 4 à 6.

6. Copolymère selon l'une des revendications précédentes,
**caractérisé en ce qu'**au moins quatre, de préférence au moins six fonctions éther sont présentes dans la chaîne principale.

7. Copolymère selon l'une des revendications 1 à 5,
**caractérisé en ce qu'**au moins deux, de préférence au moins quatre fonctions sont présentes dans la chaîne principale, choisies parmi le groupe comprenant ou consistant en fonctions ester, amide et/ou uréthane et/ou leurs combinaisons.

8. Copolymère selon l'une des revendications précédentes pour une utilisation comme support pour des principes actifs à travers une double couche lipidique.

9. Copolymère selon la revendication 8,
**caractérisé en ce que** la double couche lipidique fait partie d'une membrane biologique, de préférence d'une membrane cellulaire.

10. Copolymère selon l'une des revendications 6 à 9 pour une utilisation comme médicament.

11. Utilisation d'un copolymère selon l'une des revendications 1 à 9 pour transporter des principes actifs à travers une double couche lipidique in vitro.

12. Utilisation selon la revendication 11,
**caractérisée en ce que** la double couche lipidique fait partie d'une membrane biologique, de préférence d'une membrane cellulaire.

13. Procédé pour transporter un principe actif à travers une double couche lipidique in vitro, dans lequel un copolymère selon l'une des revendications 1 à 9 dans une solution aqueuse est fourni sur un côté de la double couche lipidique.
